# EUROPEAN PATENT SPECIFICATION

(11) **EP 3 795 169 B1**
(45) Date of publication and mention of the grant of the patent: **23.08.2023**
(21) Application number: 19797136.9
(22) Date of filing: 30.04.2019
(51) Int. Cl.: A61K 38/16, A61K 48/00, A23L 33/18, A23K 20/147, C07K 14/005, A61P 31/00, C12N 9/36

(54) **BACTERIOPHAGE-DERIVED RECOMBINANT PROTEIN HAVING ANTIMICROBIAL ACTIVITY AGAINST PATHOGENIC GRAM-NEGATIVE BACTERIA**
AUS BAKTERIOPHAGEN STAMMENDES REKOMBINANTES PROTEIN MIT ANTIMIKROBIELLER WIRKUNG GEGEN PATHOGENE GRAM-NEGATIVE BAKTERIEN
PROTÉINE RECOMBINANTE DÉRIVÉE D'UN BACTÉRIOPHAGE AYANT UNE ACTIVITÉ ANTIMICROBIENNE CONTRE DES BACTÉRIES À GRAM NÉGATIF PATHOGÈNES

(30) Priority: 03.05.2018 KR 20180051191
(43) Date of publication of application: 24.03.2021
(73) Proprietor: Kyungpook National University Industry-Academic Cooperation Foundation, Daegu 41566 (KR)
(72) Inventor: KIM, Jung Min, Daegu 42016 (KR); KIM, Shukho, Daegu 42166 (KR)
(74) Representative: Roos, Peter
(86) International application number: PCT/KR2019/005238
(87) International publication number: WO 2019/212244

(56) References cited:
- WO-A1-2013/169102
- WO-A1-2017/203471
- WO-A2-2010/123200
- CN-A- 104 830 806
- KR-B1- 100 883 132
- Mark Fenton: "Recombinant bacteriophage lysins as antibacterials", Bioengineered Bugs, 1 January 2010 (2010-01-01), pages 9-16, XP055194564, DOI: 10.4161/bbug.1.1.9818 Retrieved from the Internet: URL:http://www.ncbi.nlm.nih.gov/pmc/articl es/PMC3035150/pdf/bbug0101_0009.pdf [retrieved on 2015-06-09]
- OLIVEIRA H ET AL: "A thermostable Salmonella phage endolysin, Lys68, with broad bactericidal properties against gram-negative pathogens in presence of weak acids", PLOS ONE, PUBLIC LIBRARY OF SCIENCE, US , vol. 9, no. 12 4 December 2014 (2014-12-04), pages e108376-1, XP008182640, ISSN: 1932-6203, DOI: 10.1371/JOURNAL.PONE.0108376 Retrieved from the Internet: URL:http://journals.plos.org/plosone/artic le/file?id=10.1371/journal.pone.0108376&ty pe=printable
- LAI WING CHING BIANCA ET AL: "Bacteriophage-derived endolysins to target gram-negative bacteria", INTERNATIONAL JOURNAL OF PHARMACEUTICS, ELSEVIER, NL, vol. 589, 30 August 2020 (2020-08-30), XP086309091, ISSN: 0378-5173, DOI: 10.1016/J.IJPHARM.2020.119833 [retrieved on 2020-08-30]
- Sung Hun Kim: "Molecular epidemiological study of Salmonella enterica serovar Enteritidis and characterization of Salmonella bacteriophage SS3e", Thesis, vol. 42, 2009, pages 1-76, XP009523948,
- KIM, SUNG-HUN: "Complete genome sequence of Salmonella bacteriophage SS3e", Journal of Virology, vol. 86, no. 18s, 2012, pages 10253-10254, XP055649824,
- KIM, SHUKHO: "Characterization of a Salmonella Enteritidis bacteriophage showing broad lytic activity against Gram-negative enteric bacteria", Journal of Microbiology, 25 October 2018 (2018-10-25), pages 917-925, XP036647207,

## Description

### [Technical Field]

The present invention relates to a recombinant protein LysSS having antibacterial activity derived from bacteriophage, and more particularly, to a pharmaceutical composition for preventing or treating infectious diseases comprising the antibacterial protein as an active ingredient, an antibiotic comprising the antibacterial protein as an active ingredient, disinfectants, food additives or feed additives.

### [Background Art]

As cases where pathogenic bacteria are resistant to multi-drug antimicrobial agents and thus cannot be treated with antibiotics has increased significantly, the development of new antimicrobial agents is urgent.

As a biological resource to be noted in the development of new antimicrobial agents, bacteriophages having specific killing activity to bacteria are being studied, and attempts to use antibacterial substances possessed by bacteriophages are in progress. Bacteriophage, a virus of bacteria, infects host bacteria according to their biological life history to produce their own replica phage, and generates proteins that penetrate or degrade the bacterial cell wall during the process of killing host bacteria and the protein decomposes and breaks down the cell wall composed of peptidoglycans and kills bacteria.

Until now, the effect of killing bacteria by the antibacterial protein as expected by genetically engineered production and purification of the above bacteriophage-derived antibacterial proteins and treatment of target bacteria, but it was not as effective as conventional antibacterial agents. In particular, the effects of antibacterial proteins against gram-negative bacteria were insufficient. The reason is that when the purified antibacterial protein is treated outside the bacteria, it was confirmed that the antibacterial protein did not reach the peptidoglycan cell wall and thus did not exhibit effective antibacterial activity, due to an outer membrane of the membrane structure of gram-negative bacteria. For this reason, the use of antimicrobial proteins that degrade cell walls has been biased toward development for gram-positive bacteria, and thus, if the antimicrobial protein can effectively kill gram-negative bacteria, it will be received attention as a very promising antibacterial agent.

In addition, the reason for using bacteriophage-derived antibacterial protein produced and purified as an antibacterial agent without using the bacteriophage itself is that it is more advantageous to commercialize a protein having antibacterial activity derived from bacteriophage rather than bacteriophage itself due to stability problems and quality control problems of bacteriophage. WO 2017/203471 A1 relates to the field of antimicrobial enzymes. Sung Hun Kim, "Molecular epidemiological study of Salmonella enterica serovar Enteritidis and characterization of Salmonella bacteriophage SS3e", Thesis, vol. 42, pages 1-76, is a molecular epidemiological study of Salmonella enterica serovar Enteritidis.

Accordingly, the present invention is intended to develop as an antimicrobial agent that treats bacteria having resistance to multi-drug antimicrobial agents by using a bacteriophage-derived recombinant protein having killing activity to gram-negative bacteria.

### [Disclosure]

### [Technical Problem]

Accordingly, an object of the present invention is to provide a use of a bacteriophage-derived recombinant protein having antibacterial activity against pathogenic bacteria.

### [Technical Solution]

In order to achieve the above object, the present invention provides a pharmaceutical composition for use in preventing or treating bacterial infectious diseases comprising a protein having killing activity to pathogenic bacteria, wherein the protein comprises an amino acid sequence represented by SEQ ID NO: 1 as an active ingredient, and the pathogenic bacteria are a gram-negative bacteria selected from the group consisting of Acinetobacter baumannii, Escherichia coli, Pseudomonas aeruginosa, Salmonella enteritidis and Salmonella typhimurium.

In addition, the present invention provides an antibiotic, a disinfectant, a food additive, or a feed additive comprising a protein having killing activity to pathogenic bacteria, wherein the protein comprises an amino acid sequence represented by SEQ ID NO: 1 as an active ingredient, and the pathogenic bacteria are a gram-negative bacteria selected from the group consisting of Acinetobacter baumannii, Escherichia coli, Pseudomonas aeruginosa, Salmonella enteritidis and Salmonella typhimurium.

### [Advantageous Effects]

The bacteriophage-derived recombinant protein LysSS of the present invention exhibits killing activity to gram-negative bacteria and thus can prevent or treat infectious diseases caused by bacteria, and can be widely used in antibiotics, disinfectants, food additives, feed additives, and the like. In particular, the LysSS uses peptidoglycan, which is a component of the cell wall of bacteria, as a substrate, and exhibits bacterial killing ability due to peptidoglycan degradation. The peptidoglycan exists only in bacteria and not in humans or animals, and thus there is an advantage that LysSS of the present invention is safe because it does not affect humans and animals, and can be applied to the pharmaceutical industry, food industry, biotechnology, etc., as well as can effectively kill bacteria in a target place or a target substance without problems of resistance to multi-drug antimicrobial agents.

### [Description of Drawings]

FIG. 1 shows the amino acid sequence of LysSS, an antibacterial protein derived from bacteriophage having antibacterial activity (the underlined part is an artificially added amino acid sequence).
FIG. 2 shows the nucleotide sequence of the LysSS.
FIG. 3 shows image of SDS-PAGE analysis after protein purification of LysSS and confirming by Western analysis using a LysSS specific antibody.
FIG. 4 shows the killing activity to Salmonella by the LysSS.

### [Best Mode]

The inventors of the present invention transformed DNA encoding a recombinant protein (referred to as 'LysSS') derived from bacteriophage SS3e into E. *coli* together with a vector to express the protein, and confirmed that the protein exhibited killing activity in gram-negative bacteria and thus it can be used as a broad spectrum antimicrobial agent and completed the present invention. On the other hand, SS3e bacteriophage, the source of LysSS, can kill several gram-negative bacteria, including Salmonella, but cannot kill gram-negative bacteria such as *Acinetobacter baumannii* and *Pseudomonas aeruginosa,* whereas LysSS exhibits a broad spectrum of antimicrobial activity showing antibacterial effects on all of them.

The present invention provides a pharmaceutical composition for preventing or treating bacterial infectious diseases comprising a protein having killing activity to pathogenic bacteria, wherein the protein comprises an amino acid sequence represented by SEQ ID NO: 1 as an active ingredient.

The gene encoding the protein may be represented by SEQ ID NO: 2, but it is not limited thereto.

The protein may be derived from the bacteriophage SS3e (Accession No. KCTC 11492BP) of Siphoviridae family. The present inventors screened a novel bacteriophage having killing activity to various pathogenic bacteria from river water in the metropolitan area, and deposited the isolated bacteriophage to the Korea Human Gene Bank (KHGB) of the Korea Research Institute of Bioscience & Biotechnology (KRIBB) on March 27, 2009.

The pathogenic bacteria are gram-negative bacteria, and the gram-negative bacteria are the gram-negative bacteria selected from the group consisting of Acinetobacter baumannii, Escherichia coli, Pseudomonas aeruginosa, Salmonella enteritidis and Salmonella typhimurium.

The gram-negative bacteria are bacteria that are stained red when stained by the gram staining method, and are characterized in that a peptidoglycan cell wall is formed between an inner cytoplasmic membrane and an outer membrane like a sandwich. Gram-negative bacteria are spread all over the world and can live in almost any living environment.

The protein of the present invention uses peptidoglycan, which is a component of the cell wall of bacteria, as a substrate to degrade and disrupt the cell wall, thereby killing the bacteria. The peptidoglycan exists only in bacteria and not in humans or animals, and thus there is an advantage that protein of the present invention is safe because it does not affect humans and animals, and can be applied to the pharmaceutical industry, food industry, biotechnology, etc., as well as can effectively kill bacteria in a target place or a target substance without problems of resistance to multi-drug antimicrobial agents.

The pathogenic bacteria cause various diseases, and the bacterial infectious diseases may be selected from the group consisting of sepsis, pneumonia, food poisoning, dysentery, Pseudomonas aeruginosa infection, impetigo, purulent disease, acute dermatitis, bacteremia, endocarditis and enteritis but they are not limited thereto.

As used herein, the term 'treatment' refers to the prevention, inhibition and alleviation of infectious diseases caused by pathogenic bacteria.

When the composition of the present invention is a pharmaceutical composition, for administration, it may include a pharmaceutically acceptable carrier, excipient or diluent in addition to the above-described active ingredients. Examples of the carrier, excipient and diluent include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methylhydroxybenzoate, propylhydroxybenzoate, talc, magnesium stearate and mineral oils.

The pharmaceutical compositions of the present invention can be formulated and used in the form of oral formulations such as powders, granules, tablets, capsules, suspensions, emulsions, syrups, aerosols, external preparations, suppositories, or sterile injectable solutions according to a conventional method. In detail, when formulated, it may be prepared using diluents or excipients such as fillers, weighting agents, binders, wetting agents, disintegrants and surfactants that are commonly used. Solid preparations for oral administration include tablets, pills, powders, granules, capsules, and the like, but they are not limited thereto. Such a solid preparation may be prepared by mixing at least one excipient, for example, starch, calcium carbonate, sucrose, lactose, gelatin, etc. in addition to the active ingredient. Further, in addition to simple excipients, lubricants such as magnesium stearate and talc may also be used. It can be prepared by adding various excipients such as wetting agents, sweetening agents, fragrances, preservatives, and the like, in addition to liquids and liquid paraffins for oral use. Formulations for parenteral administration include sterile aqueous solutions, non-aqueous solvents, suspensions, emulsions, lyophilized formulations, and suppositories. As the non-aqueous solvent and suspending agent, propylene glycol, polyethylene glycol, vegetable oil such as olive oil, injectable ester such as ethyl oleate, and the like may be used. As a base for suppositories, witepsol, macrosol, Tween 61, cacao butter, laurin, glycerogelatin, and the like may be used.

A suitable dosage of the pharmaceutical composition of the present invention varies depending on the condition and weight of the patient, the severity of the disease, the form of the drug, and the time, but can be appropriately selected by a person skilled in the art. Thus, the daily dosage of the pharmaceutically acceptable salt is preferably 0.001 mg/kg to 50 mg/kg, and may be administered once to several times a day as necessary.

In addition, the present invention provides a cosmetic antimicrobial agent and a pharmaceutical antibiotic comprising a protein having killing activity to pathogenic bacteria, wherein the protein comprises an amino acid sequence represented by SEQ ID NO: 1 as an active ingredient.

Cosmetics contain oil or water as their main components, and because there are many combinations of glycerin or sorbitol as carbon sources of microorganisms, amino acid derivatives and proteins as nitrogen sources, it is easy for microorganisms such as bacteria. In addition, it can be said that the risk of contamination by microorganisms is much greater because the period of use is very long compared to that of food. It is essential to add an antibacterial agent to protect cosmetics for a long time from discoloration or deodorant caused by microbial contamination due to use.

The protein of the present invention has excellent ability to kill a wide range of bacteria compared to conventional antimicrobial agents. If the protein is used as an antimicrobial agent, unlike conventional antimicrobial agents, it has the advantage of not inducing tolerance or resistance of bacteria to provide an antibiotic material having a longer life cycle compared to the conventional antibiotic material. While most of the antibiotics face increased resistance, the range of use decreases, whereas the antimicrobial agent comprising the protein of the present invention as an active ingredient can fundamentally solve the problem of resistance to antibacterial agents, thereby increasing the product lifespan as an antimicrobial agent.

Therefore, an antibiotic comprising the protein of the present invention having killing activity to pathogenic bacteria as an active ingredient can be usefully used as an antibiotic having excellent antibacterial, bactericidal and antiseptic effects. The term "antibiotic" as used herein collectively refers to preservatives, fungicides and antibacterial agents.

In addition, the present invention provides a disinfectant comprising a protein having killing activity to pathogenic bacteria, wherein the protein comprises an amino acid sequence represented by SEQ ID NO: 1 as an active ingredient.

The disinfectant comprising the protein of the present invention having a wide range of killing activity to bacteria as an active ingredient can be usefully used as a disinfectant for hospitals and health care to prevent hospital infection, and also as a disinfectant for general life, food and cooking places and facilities, livestock housing in the livestock industry.

In addition, the present invention provides a food additive comprising a protein having killing activity to pathogenic bacteria, wherein the protein comprises an amino acid sequence represented by SEQ ID NO: 1 as an active ingredient.

Furthermore, the present invention provides a feed additive comprising a protein having killing activity to pathogenic bacteria, wherein the protein comprises an amino acid sequence represented by SEQ ID NO: 1 as an active ingredient.

The protein of the present invention can be used as an additive for livestock feed and drinking water for livestock for the purpose of preventing or treating bacterial infections and can improve or maintain animal feed intake, growth, feed efficiency, survival rate, feeding condition, production capacity, etc.

Hereinafter, the present invention will be described in more detail through examples. These examples are only intended to illustrate the present invention in more detail, and it will be apparent to those skilled in the art that the scope of the present invention is not limited by these examples according to the gist of the present invention.

### Example 1: Production and purification of bacteriophage-derived recombinant protein LysSS

To obtain a fragment containing open reading frame (open reading frame 25; ORF25) of the bacteriophage SS3e (Accession No. KCTC 11492BP) of Siphoviridae family having specific killing activity to Salmonella, gDNA of the bacteriophage SS3e was extracted. A polymerase chain reaction (PCR) was performed using the gDNA as a template, and the primers used were as follows: SS3e25-F: GGGAATTCCATATGTCAAACCGAAACATTAG, SS3e25-R: ATCCGCTCGAGCTTTGCTTCGCGGCCGATG. The PCR fragment was obtained by eluting a band of a desired size after electrophoresis on an agarose gel.

The PCR fragment was digested by treatment with restriction enzymes Ndel and Xhol, and then ligated with an expression vector (pET21a) digested with the same enzyme. When E. *coli* BL21 (DE3) was transformed (LysSS-pET21a) with the prepared vector, the culture was performed in the LB liquid medium to which 100 µg/ml of ampicillin was added until the absorbance of the bacteria reached 0.6 (600 nm wavelength). Next, IPTG (isopropyl β-D-1-thiogalactopyranoside) was added so that the final concentration was 0.1 mM, followed by incubation at 18 °C for 16 hours to induce protein expression.

Thereafter, after harvesting the bacteria, the bacteria were disrupted with a lysis buffer [50 mM Tris-Cl (pH 8.0), 200 mM NaCl] using an ultrasonicator. The disrupted bacterial lysate was centrifuged to obtain a supernatant, and injected into a Ni-NTA column, and LysSS proteins tagged with six histidines at the C-terminus were purified using an elution buffer [500 mM imidazole, 50 mM Tris-Cl (pH 8.0), 200 mM NaCl].

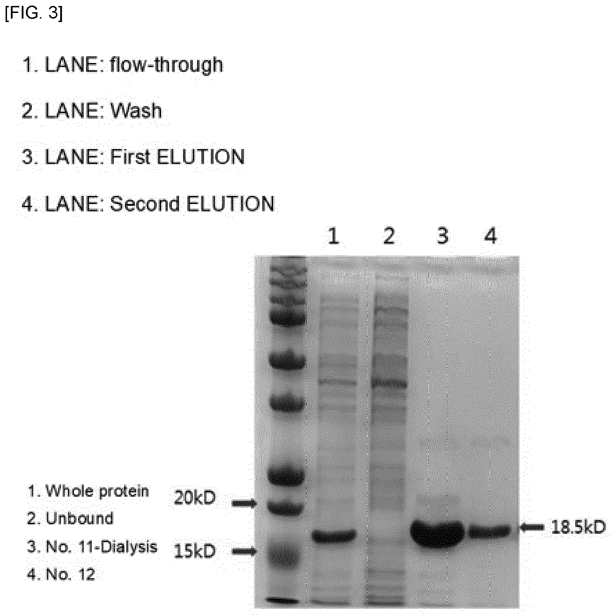

In order to identify the purified LysSS protein, SDS-PAGE (12%) and staining with Coomassie blue was performed and as a result, LysSS protein was confirmed at 18.5 kD as shown in FIG. 3 and it was confirmed that the 18.5 kD protein was 6xHis tagged LysSS by Western analysis using anti-6xHis monoclonal antibody.

In FIG. 3A, M is a protein size marker, 1 is a flow-through of the sample initially flown from the Ni-NTA column, 2 is a wash sample, 3-5 is samples of three fractions harvested from LysSS, and FIG. 3B shows Western test results using anti-6xHis monoclonal antibody for LysSS and M is a protein size marker, 1 is the fraction sample of harvested LysSS.

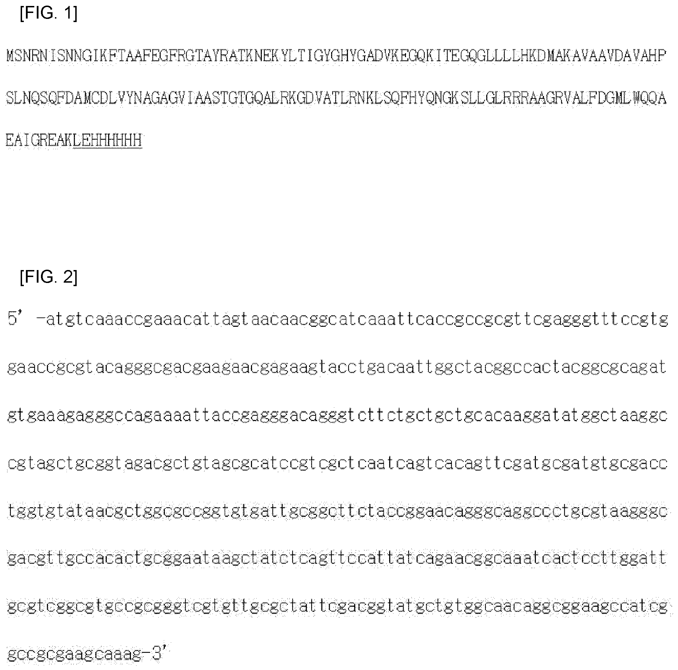

As shown in FIG. 1, the amino acid sequence and number of LysSS is composed of 170 amino acids including histidine tag, has a total size of 18.5 kDa and has 60-97% homology to the lysozyme of Salmonella bacteriophage (NCBI Blast Search). The gene coding sequence is 510 bp as shown in FIG. 2.

### Example 2: Analysis of bacterial killing activity of LysSS

As for gram-negative bacteria *Salmonella enteritidis* 2 species, *Salmonella typhimurium* 2 species, *Pseudomonas aeruginosa* 1 species, *Acinetobacter baumannii* 1 species, *Klebsiella pneumoniae* 2 species, *Escherichia coli* 2 species, and gram-positive *Staphylococcus aureus* 2 species, the bacterial killing ability of LysSS was performed as follows (Table 1). Each bacterium was prepared so that the number of bacteria was 5 × 10⁵/mL CFU using a buffer [50 mM Tris-Cl (pH 8.0), 200 mM NaCl], and the purified LysSS protein was added to be 0.25, 0.5, 1.0 mg/ mL, respectively and reacted at 35 °C for 16 hours and then the bacterial turbidity was observed. After spreading on MAC agar (MacConkey agar; MCA) medium or LB agar (Luria Bertani agar: LBA) medium and incubating for 16 hours, the number of bacteria was measured.

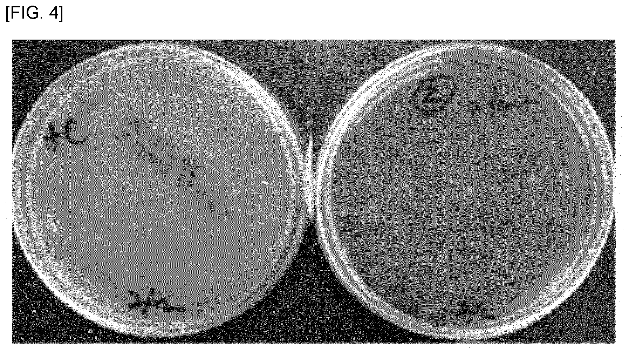

As a result, as shown in FIG. 4, growth of bacteria was observed in both the test tube and the well plate of the control group (-LysSS), whereas the test group (+LysSS) showed a transparent liquid in which no bacterial growth was observed. In the solid medium, about 5 × 10⁶ CFU bacteria were observed in the control group (-LysSS), whereas only Salmonella bacteria of less than 10 CFU survived in the test group treated with LysSS and had 99.99999% of killing activity to Salmonella.

Table 1 below shows the strains used in the bacterial killing ability test of LysSS and the minimum inhibitory concentration (MIC) of LysSS for each test strain.

**[Table 1]**

| Bacterial species | Classification | Antimicrobial effect of LysSS (MIC^{*}, mg/mL) |
|---|---|---|
| *Salmonella enteritidis* KNIH 14771 | gram-negative bacteria | Yes (1.0) |
| *Salmonella enteritidis* Phage Type 4 | gram-negative bacteria | Yes (0.5) |
| *Salmonella typhimurium* KNIH 14772 | gram-negative bacteria | Yes (0.5) |
| *Salmonella typhimurium* Phage Type DT104 | gram-negative bacteria | Yes (1.0) |
| *Pseudomonas aeruginosa* PAO1 | gram-negative bacteria | Yes (0.5) |
| *Acinetobacter baumannii* ATCC 17978 | gram-negative bacteria | Yes (0.5) |
| *Klebsiella pneumoniae* KCTC 2208 | gram-negative bacteria | No |
| *Klebsiella pneumoniae* KNU 05K650 | gram-negative bacteria | No |
| *Escherichia coli* ATCC 25922 | gram-negative bacteria | Yes (0.5) |
| *Escherichia coli* DH5α | gram-negative bacteria | Yes (0.25) |
| *Staphylococcus aureus* ATCC 29213 | gram-positive bacteria | No |
| *Staphylococcus aureus* ATCC 25923 | gram-positive bacteria | No |

| | | |
|---|---|---|
| *MIC: minimum inhibitory concentration | | |

Meanwhile, examples of preparations using the protein of the present invention are exemplified below, but this is not intended to limit the present invention, but to describe in detail.

### [Formulation Example 1: Preparation of powder]

Recombinant protein LysSS 300 mg
Lactose 100 mg
Talc 10 mg

The above ingredients are mixed and filled in an airtight cloth to prepare a powder.

### [Formulation Example 2: Preparation of tablets]

Recombinant protein LysSS 300 mg
Corn starch 100 mg
Lactose 100 mg
Magnesium stearate 2 mg

After mixing the above ingredients, tablets are prepared by compressing according to a conventional tablet preparation method.

### [Formulation Example 3: Preparation of capsules]

Recombinant protein LysSS 300 mg
Crystalline cellulose 3 mg
Lactose 14.8 mg
Magnesium stearate 0.2 mg

According to a conventional capsule preparation method, the above ingredients are mixed and filled into gelatin capsules to prepare a capsule.

### [Formulation Example 4: Preparation of Injection formulation]

Recombinant protein LysSS 300 mg
Mannitol 180 mg
Sterile distilled water for injection 2974 mg
Na₂HPO₄•2H₂O 26 mg

According to a conventional injection preparation method, it is prepared with the above ingredients per ampoule (2)

### [Formulation Example 5: Preparation of liquid formulation]

Recombinant protein LysSS 300 mg
Isomerized sugar 10 g
Mannitol 5 g
Purified water appropriate amount

According to a conventional preparation method of liquid formulation, each ingredient is added to purified water to dissolve it, lemon zest is added and the above ingredients are mixed, purified water is added to adjust the total amount to 100, then filled in a brown bottle for sterilization to prepare liquid formulation.

### [Formulation Example 6: Preparation of food additives]

A milk composition according to the present invention was prepared by adding 1% (w/v) of the recombinant protein LysSS of the present invention to 200 mL of commercially available S-manufactured milk.

### [Formulation Example 7: Preparation of feed additive]

A feed additive was prepared according to the method for producing a feed additive by mixing 100 g of the recombinant protein LysSS of the present invention and an appropriate amount of an excipient.

### [Formulation Example 8: Feed Preparation]

A feed was prepared according to a conventional feed preparation method by mixing recombinant protein of the present invention LysSS of 50 g, mushroom medium of 200 g, wheat brp of 30 g, beet pulp of 50 g, rice DDGS (Distillers Dried Grains with Solubles) of 220 g, corn flakes of 200 g, whole soybean of 40 g, starch pulp of 100 g, corn silage of 200 g, corn cob of 180 g, bean-curd dregs of 400 g, ryegrass of 323 g, geolite of 14 g and tapioca of 40 g.

While the present invention has been particularly described with reference to specific embodiments thereof, it is apparent that this specific description is only a preferred embodiment and that the scope of the present invention is not limited thereby to those skilled in the art. That is, the practical scope of the present invention is defined by the appended claims.

## Claims

1. A pharmaceutical composition for use in preventing or treating bacterial infectious diseases comprising a protein having killing activity to pathogenic bacteria, wherein the protein comprises an amino acid sequence represented by SEQ ID NO: 1 as an active ingredient, and the pathogenic bacteria are a gram-negative bacteria selected from the group consisting of *Acinetobacter baumannii, Escherichia coli, Pseudomonas aeruginosa, Salmonella enteritidis* and *Salmonella typhimurium.*

2. The pharmaceutical composition for use in preventing or treating bacterial infectious diseases of claim 1, wherein a gene encoding the protein is represented by SEQ ID NO: 2.

3. The pharmaceutical composition for use in preventing or treating bacterial infectious diseases of claim 1, wherein the protein is derived from bacteriophage SS3e.

4. The pharmaceutical composition for use in preventing or treating bacterial infectious diseases of claim 1, wherein the bacterial infectious disease is selected from the group consisting of sepsis, pneumonia, food poisoning, dysentery, Pseudomonas aeruginosa infection, impetigo, purulent disease, acute dermatitis, bacteremia, endocarditis and enteritis.

5. An antibiotic comprising a protein having killing activity to pathogenic bacteria, wherein the protein comprises an amino acid sequence represented by SEQ ID NO: 1 as an active ingredient, and the pathogenic bacteria are a gram-negative bacteria selected from the group consisting of *Acinetobacter baumannii, Escherichia coli, Pseudomonas aeruginosa, Salmonella enteritidis* and *Salmonella typhimurium.*

6. A disinfectant comprising a protein having killing activity to pathogenic bacteria, wherein the protein comprises an amino acid sequence represented by SEQ ID NO: 1 as an active ingredient, and the pathogenic bacteria are a gram-negative bacteria selected from the group consisting of *Acinetobacter baumannii, Escherichia coli, Pseudomonas aeruginosa, Salmonella enteritidis* and *Salmonella typhimurium.*

7. A food additive comprising a protein having killing activity to pathogenic bacteria, wherein the protein comprises an amino acid sequence represented by SEQ ID NO: 1 as an active ingredient, and the pathogenic bacteria are a gram-negative bacteria selected from the group consisting of *Acinetobacter baumannii, Escherichia coli, Pseudomonas aeruginosa, Salmonella enteritidis* and *Salmonella typhimurium.*

8. A feed additive comprising a protein having killing activity to pathogenic bacteria, wherein the protein comprises an amino acid sequence represented by SEQ ID NO: 1 as an active ingredient, and the pathogenic bacteria are a gram-negative bacteria selected from the group consisting of *Acinetobacter baumannii, Escherichia coli, Pseudomonas aeruginosa, Salmonella enteritidis* and *Salmonella typhimurium.*

## Patentansprüche

1. Pharmazeutische Zusammensetzung zur Verwendung bei der Vorbeugung oder Behandlung von bakteriellen Infektionskrankheiten, umfassend ein Protein mit Abtötungsaktivität gegenüber pathogenen Bakterien, wobei das Protein eine durch SEQ ID NO: 1 dargestellte Aminosäuresequenz als einen Wirkstoff umfasst und die pathogenen Bakterien ein gramnegatives Bakterium sind, ausgewählt aus der Gruppe bestehend aus *Acinetobacter baumannii, Escherichia coli, Pseudomonas aeruginosa, Salmonella enteritidis* und *Salmonella typhimurium.*

2. Pharmazeutische Zusammensetzung zur Verwendung bei der Vorbeugung oder Behandlung bakterieller Infektionskrankheiten nach Anspruch 1, wobei ein das Protein kodierendes Gen durch SEQ ID NO: 2 dargestellt wird.

3. Pharmazeutische Zusammensetzung zur Verwendung bei der Vorbeugung oder Behandlung von bakteriellen Infektionskrankheiten nach Anspruch 1, wobei das Protein vom Bakteriophagen SS3e abgeleitet ist.

4. Pharmazeutische Zusammensetzung zur Verwendung bei der Vorbeugung oder Behandlung bakterieller Infektionskrankheiten nach Anspruch 1, wobei die bakterielle Infektionskrankheit ausgewählt ist aus der Gruppe bestehend aus Sepsis, Lungenentzündung, Lebensmittelvergiftung, Dysenterie, Pseudomonas aeruginosa-Infektion, Impetigo, eitrige Erkrankung, akute Dermatitis, Bakteriämie, Endokarditis und Enteritis.

5. Antibiotikum, umfassend ein Protein mit Abtötungsaktivität gegenüber pathogenen Bakterien, wobei das Protein eine durch SEQ ID NO: 1 dargestellte Aminosäuresequenz als einen Wirkstoff umfasst und die pathogenen Bakterien gramnegative Bakterien sind, ausgewählt aus der Gruppe bestehend aus *Acinetobacter baumannii, Escherichia coli, Pseudomonas aeruginosa, Salmonella enteritidis* und *Salmonella typhimurium.*

6. Desinfektionsmittel, umfassend ein Protein mit Abtötungsaktivität gegenüber pathogenen Bakterien, wobei das Protein eine durch SEQ ID NO: 1 dargestellte Aminosäuresequenz als einen Wirkstoff umfasst und die pathogenen Bakterien gramnegative Bakterien sind, ausgewählt aus der Gruppe bestehend aus *Acinetobacter baumannii, Escherichia coli, Pseudomonas aeruginosa, Salmonella enteritidis* und *Salmonella typhimurium.*

7. Nahrungsmittelzusatz, umfassend ein Protein mit Abtötungsaktivität gegenüber pathogenen Bakterien, wobei das Protein eine durch SEQ ID NO: 1 dargestellte Aminosäuresequenz als einen Wirkstoff umfasst und die pathogenen Bakterien gramnegative Bakterien sind, ausgewählt aus der Gruppe bestehend aus *Acinetobacter baumannii, Escherichia coli, Pseudomonas aeruginosa, Salmonella enteritidis* und *Salmonella typhimurium.*

8. Futtermittelzusatz, umfassend ein Protein mit Abtötungsaktivität gegenüber pathogenen Bakterien, wobei das Protein eine durch SEQ ID NO: 1 dargestellte Aminosäuresequenz als einen Wirkstoff umfasst und die pathogenen Bakterien gramnegative Bakterien sind, die aus der Gruppe bestehend aus *Acinetobacterium baumannii, Escherichia coli* und *Salmonella enterici* ausgewählt sind.

## Revendications

1. Composition pharmaceutique pour utilisation dans la prévention ou le traitement des maladies infectieuses bactériennes comprenant une protéine ayant une activité de destruction des bactéries pathogènes, dans laquelle la protéine comprend une séquence d'acides aminés représentée par SEQ ID NO : 1 en tant qu'ingrédient actif, et les bactéries pathogènes sont des bactéries gram-négatives choisies parmi le groupe constitué *d'Acinetobacter baumannii, Escherichia coli, Pseudomonas aeruginosa, Salmonella enteritidis* et *Salmonella typhimurium.*

2. Composition pharmaceutique pour utilisation dans la prévention ou le traitement des maladies infectieuses bactériennes de la revendication 1, dans laquelle un gène codant la protéine est représenté par SEQ ID NO : 2.

3. Composition pharmaceutique pour utilisation dans la prévention ou le traitement des maladies infectieuses bactériennes de la revendication 1, dans laquelle la protéine est dérivée du bactériophage SS3e.

4. Composition pharmaceutique pour utilisation dans la prévention ou le traitement des maladies infectieuses bactériennes de la revendication 1, dans laquelle la maladie infectieuse bactérienne est choisie parmi le groupe constitué par la septicémie, la pneumonie, l'intoxication alimentaire, la dysenterie, l'infection à Pseudomonas aeruginosa, l'impétigo, la maladie purulente, la dermatite aiguë, la bactériémie, l'endocardite et l'entérite.

5. Antibiotique comprenant une protéine ayant une activité de destruction des bactéries pathogènes, dans lequel la protéine comprend une séquence d'acides aminés représentée par SEQ ID NO : 1 en tant qu'ingrédient actif, et les bactéries pathogènes sont des bactéries gram-négatives choisies parmi le groupe constitué *d'Acinetobacter baumannii, Escherichia coli, Pseudomonas aeruginosa, Salmonella enteritidis* et *Salmonella typhimurium.*

6. Désinfectant comprenant une protéine ayant une activité de destruction des bactéries pathogènes, dans lequel la protéine comprend une séquence d'acides aminés représentée par SEQ ID NO : 1 en tant qu'ingrédient actif, et les bactéries pathogènes sont des bactéries gram-négatives choisies parmi le groupe constitué par *Acinetobacter baumannii, Escherichia coli, Pseudomonas aeruginosa, Salmonella enteritidis* et *Salmonella typhimurium.*

7. Additif alimentaire comprenant une protéine ayant une activité de destruction des bactéries pathogènes, dans lequel la protéine comprend une séquence d'acides aminés représentée par SEQ ID NO : 1 en tant qu'ingrédient actif, et les bactéries pathogènes sont des bactéries gram-négatives choisies parmi le groupe constitué *d'Acinetobacter baumannii, Escherichia coli, Pseudomonas aeruginosa, Salmonella enteritidis* et *Salmonella typhimurium.*

8. Additif fourrager comprenant une protéine ayant une activité de destruction des bactéries pathogènes, dans lequel la protéine comprend une séquence d'acides aminés représentée par SEQ ID NO : 1 en tant qu'ingrédient actif, et les bactéries pathogènes sont des bactéries gram-négatives choisies parmi le groupe constitué *d'Acinetobacter baumannii, Escherichia coli, Pseudomonas aeruginosa, Salmonella enteritidis* et *Salmonella typhimurium.*
